# EUROPEAN PATENT APPLICATION

(11) **EP 4 390 952 A1**
(43) Date of publication of application: **26.06.2024**
(21) Application number: 22875764.7
(22) Date of filing: 08.09.2022
(51) Int. Cl.: G16H 20/10, C12M 1/00

(54) **PHARMACEUTICAL ASSISTANCE DEVICE, METHOD OF OPERATING PHARMACEUTICAL ASSISTANCE DEVICE, AND PROGRAM FOR OPERATING PHARMACEUTICAL ASSISTANCE DEVICE**

(30) Priority: 29.09.2021 JP 2021159999
(71) Applicant: FUJIFILM Corporation, Tokyo 106-8620 (JP)
(72) Inventor: LI, Yuanzhong, Ashigarakami-gun, Kanagawa 258-8577 (JP)
(74) Representative: Hoffmann Eitle
(86) International application number: PCT/JP2022/033704
(87) International publication number: WO 2023/053892

(57) **Abstract**

Provided is a pharmaceutical support device including a processor. The processor is configured to: acquire protein information related to a protein included in a biopharmaceutical to be preserved and a plurality of prescription information items respectively related to prescriptions of a plurality of types of candidate preservation solutions which are candidates for a preservation solution for the biopharmaceutical to be preserved; derive a plurality of feature amounts respectively related to preservation stability of the plurality of types of candidate preservation solutions on the basis of the protein information and the prescription information items; input the feature amount to a first machine learning model such that a first score indicating the preservation stability of the candidate preservation solution is output from the first machine learning model; acquire measurement data indicating preservation stability measured by an actual test for a selected candidate preservation solution selected from the plurality of types of candidate preservation solutions on the basis of the first score; and input the measurement data and a feature amount of the selected candidate preservation solution among the plurality of derived feature amounts to a second machine learning model such that a second score indicating preservation stability of the selected candidate preservation solution is output from the second machine learning model.

## Description

### BACKGROUND OF THE INVENTION

### 1. Field of the Invention

The technology of the present disclosure relates to a pharmaceutical support device, a method for operating a pharmaceutical support device, and a program for operating a pharmaceutical support device.

### 2. Description of the Related Art

In recent years, biopharmaceuticals have attracted attention due to high drug efficacy and low side effects. The biopharmaceutical has, for example, a protein, such as interferon or an antibody, as an active ingredient. The biopharmaceutical is preserved in a preservation solution. It is important to prescribe a preservation solution (also referred to as a formulation prescription) suitable for the biopharmaceutical in order to stably maintain the quality of the biopharmaceutical.

The preservation solution includes a buffer solution, an additive, and a surfactant. The prescription of the preservation solution is, for example, the type and concentration of each of the buffer solution, the additive, and the surfactant and a hydrogen ion exponent (Potential of Hydrogen (pH) value) of the preservation solution.

In the related art, in a case where the prescription of the preservation solution is determined, a plurality of types of preservation solutions are prepared, for example, while changing a combination of the buffer solution, the additive, and the surfactant, and the preservation stability of each preservation solution is checked by an actual test. However, it takes a lot of time and effort to perform this operation. Therefore, for example, as in WO2021/041384A and "Theresa K. Cloutier, etc., Machine Learning Models of Antibody-Excipient Preferential Interactions for Use in Computational Formulation Design, Mol. Pharmaceutics, 17, 9, 3589-3599, 2020." (hereinafter, referred to as Document 1), a technique has been proposed that predicts preservation stability of a preservation solution on the basis of information of a protein in a biopharmaceutical or information of an additive in the preservation solution, using a molecular dynamics (MD) method or a machine learning model, without performing any test.

### SUMMARY OF THE INVENTION

In the technique disclosed in WO2021/041384A and Document 1, the preservation stability of the preservation solution is not checked by the actual test. Therefore, there is a risk that a prediction result that is far from the original preservation stability will be obtained. However, it is not advisable to check the preservation stability of all of a plurality of types of preservation solutions with the actual test from the viewpoint of efficiency as described above. Therefore, there is a demand for a method that can efficiently predict preservation stability of a preservation solution with high accuracy.

An embodiment according to the technology of the present disclosure provides a pharmaceutical support device, a method for operating a pharmaceutical support device, and a program for operating a pharmaceutical support device that can efficiently predict preservation stability of a preservation solution with high accuracy.

According to the present disclosure, there is provided a pharmaceutical support device comprising a processor. The processor is configured to: acquire protein information related to a protein included in a biopharmaceutical to be preserved and a plurality of prescription information items respectively related to prescriptions of a plurality of types of candidate preservation solutions which are candidates for a preservation solution for the biopharmaceutical to be preserved; derive a plurality of feature amounts respectively related to preservation stability of the plurality of types of candidate preservation solutions on the basis of the protein information and the prescription information items; input the feature amount to a first machine learning model such that a first score indicating the preservation stability of the candidate preservation solution is output from the first machine learning model; acquire measurement data indicating preservation stability measured by an actual test for a selected candidate preservation solution selected from the plurality of types of candidate preservation solutions on the basis of the first score; and input the measurement data and a feature amount of the selected candidate preservation solution among the plurality of derived feature amounts to a second machine learning model such that a second score indicating preservation stability of the selected candidate preservation solution is output from the second machine learning model.

Preferably, the protein information includes at least one of an amino acid sequence of the protein or a three-dimensional structure of the protein.

Preferably, the prescription information includes a type and concentration of each of a buffer solution, an additive, and a surfactant included in the candidate preservation solution and a hydrogen ion exponent of the candidate preservation solution.

Preferably, the processor is configured to derive the feature amount using a molecular dynamics method.

Preferably, the feature amount includes at least one of a solvent accessible surface area, a spatial aggregation propensity, or a space charge map of the protein.

Preferably, the feature amount includes an indicator showing compatibility between the protein and the additive included in the candidate preservation solution.

Preferably, the measurement data includes at least one of aggregation analysis data of sub-visible particles of the protein in the selected candidate preservation solution, analysis data of the protein in the selected candidate preservation solution by a dynamic light scattering method, analysis data of the protein in the selected candidate preservation solution by size exclusion chromatography, or analysis data of the protein in the selected candidate preservation solution by differential scanning calorimetry.

Preferably, the processor is configured to: determine ranking of the candidate preservation solutions on the basis of a plurality of the first scores output for the plurality of types of candidate preservation solutions; and present a determination result of the ranking.

Preferably, the processor is configured to: output a plurality of the second scores for a plurality of types of the selected candidate preservation solutions; determine ranking of the selected candidate preservation solutions on the basis of the plurality of second scores; and present a determination result of the ranking.

Preferably, the processor is configured to: input prescription information of the selected candidate preservation solution among the plurality of prescription information items to the second machine learning model, in addition to the measurement data and the feature amount.

Preferably, the protein is an antibody.

According to the present disclosure, there is provided a method for operating a pharmaceutical support device. The method comprises: acquiring protein information related to a protein included in a biopharmaceutical to be preserved and a plurality of prescription information items respectively related to prescriptions of a plurality of types of candidate preservation solutions which are candidates for a preservation solution for the biopharmaceutical to be preserved; deriving a plurality of feature amounts respectively related to preservation stability of the plurality of types of candidate preservation solutions on the basis of the protein information and the prescription information items; inputting the feature amount to a first machine learning model such that a first score indicating the preservation stability of the candidate preservation solution is output from the first machine learning model; acquiring measurement data indicating preservation stability measured by an actual test for a selected candidate preservation solution selected from the plurality of types of candidate preservation solutions on the basis of the first score; and inputting the measurement data and a feature amount of the selected candidate preservation solution among the plurality of derived feature amounts to a second machine learning model such that a second score indicating preservation stability of the selected candidate preservation solution is output from the second machine learning model.

According to the present disclosure, there is provided a program for operating a pharmaceutical support device. The program causes a computer to execute a process comprising: acquiring protein information related to a protein included in a biopharmaceutical to be preserved and a plurality of prescription information items respectively related to prescriptions of a plurality of types of candidate preservation solutions which are candidates for a preservation solution for the biopharmaceutical to be preserved; deriving a plurality of feature amounts respectively related to preservation stability of the plurality of types of candidate preservation solutions on the basis of the protein information and the prescription information items; inputting the feature amount to a first machine learning model such that a first score indicating the preservation stability of the candidate preservation solution is output from the first machine learning model; acquiring measurement data indicating preservation stability measured by an actual test for a selected candidate preservation solution selected from the plurality of types of candidate preservation solutions on the basis of the first score; and inputting the measurement data and a feature amount of the selected candidate preservation solution among the plurality of derived feature amounts to a second machine learning model such that a second score indicating preservation stability of the selected candidate preservation solution is output from the second machine learning model.

According to the technology of the present disclosure, it is possible to provide a pharmaceutical support device, a method for operating a pharmaceutical support device, and a program for operating a pharmaceutical support device that can efficiently predict preservation stability of a preservation solution with high accuracy.

### BRIEF DESCRIPTION OF THE DRAWINGS

Fig. 1 is a diagram illustrating a pharmaceutical support server and an operator terminal.
Fig. 2 is a diagram illustrating an aspect in which a selected candidate preservation solution predicted to have high preservation stability is selected from a plurality of types of candidate preservation solutions.
Fig. 3 is a diagram illustrating antibody information.
Fig. 4 is a diagram illustrating prescription information.
Fig. 5 is a diagram illustrating a series of flows of preparation of the selected candidate preservation solution, addition of an antibody, a stress test, and measurement of measurement data, prescription information, and measurement data.
Fig. 6 is a block diagram illustrating a computer constituting the pharmaceutical support server.
Fig. 7 is a block diagram illustrating processing units of a CPU of the pharmaceutical support server.
Fig. 8 is a diagram illustrating a process of a feature amount derivation unit.
Fig. 9 is a diagram illustrating a process of a first prediction unit.
Fig. 10 is a diagram illustrating processes of the first prediction unit and a delivery control unit.
Fig. 11 is a diagram illustrating a process of a second prediction unit.
Fig. 12 is a diagram illustrating processes of the second prediction unit and the delivery control unit.
Fig. 13 is a diagram illustrating a first information input screen.
Fig. 14 is a diagram illustrating a first prediction result display screen.
Fig. 15 is a diagram illustrating a second information input screen.
Fig. 16 is a diagram illustrating a second prediction result display screen.
Fig. 17 is a flowchart illustrating a processing procedure of the pharmaceutical support server.
Fig. 18 is a flowchart illustrating the processing procedure of the pharmaceutical support server.
Fig. 19 is a diagram illustrating a procedure according to the related art which selects a preservation solution suitable for a biopharmaceutical to be preserved from a plurality of types of candidate preservation solutions.
Fig. 20 is a diagram illustrating a procedure according to this example which selects a selected candidate preservation solution predicted to have high preservation stability from a plurality of types of candidate preservation solutions.
Fig. 21 is a diagram illustrating a procedure according to this example which selects a preservation solution suitable for a biopharmaceutical to be preserved from a plurality of types of selected candidate preservation solutions.
Fig. 22 is a diagram illustrating a process of a second prediction unit according to a second embodiment.

### DESCRIPTION OF THE PREFERRED EMBODIMENTS

### [First Embodiment]

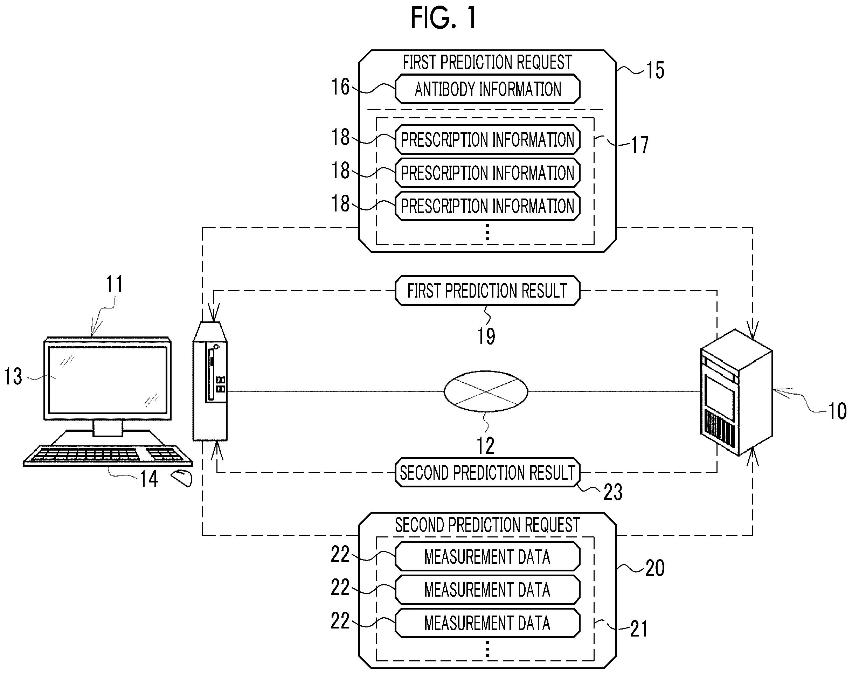

As illustrated in Fig. 1 as an example, a pharmaceutical support server 10 is connected to an operator terminal 11 through a network 12. The pharmaceutical support server 10 is an example of a "pharmaceutical support device" according to the technology of the present disclosure. The operator terminal 11 is installed, for example, in a pharmaceutical facility and is operated by an operator, such as a pharmacist, who is involved in the production of biopharmaceuticals in the pharmaceutical facility. The operator terminal 11 has a display 13 and an input device 14 such as a keyboard and a mouse. The network 12 is, for example, a wide area network (WAN) such as the Internet or a public communication network. In addition, in Fig. 1, only one operator terminal 11 is connected to the pharmaceutical support server 10. However, in practice, a plurality of operator terminals 11 in a plurality of pharmaceutical facilities are connected to the pharmaceutical support server 10.

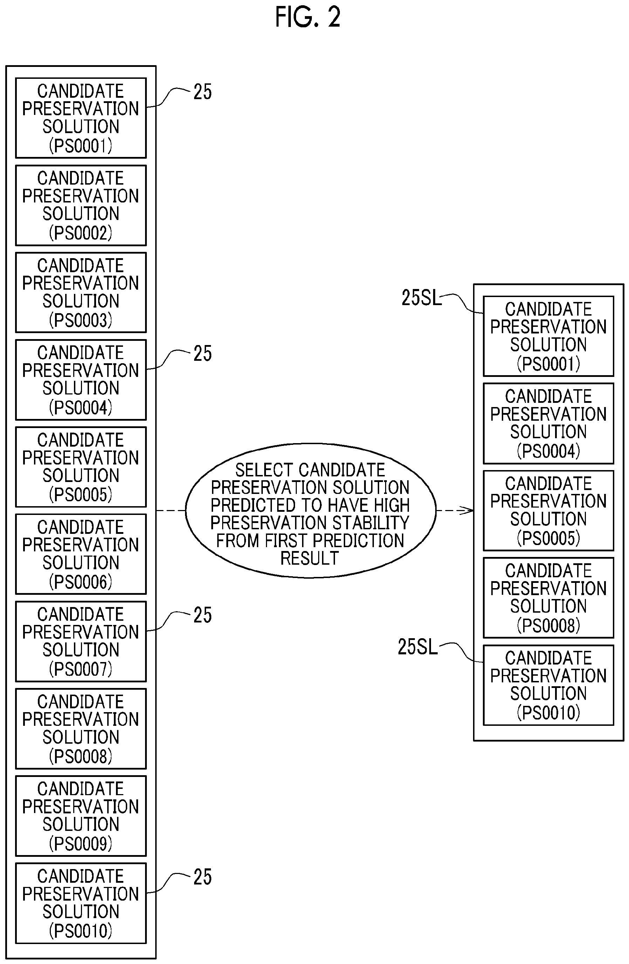

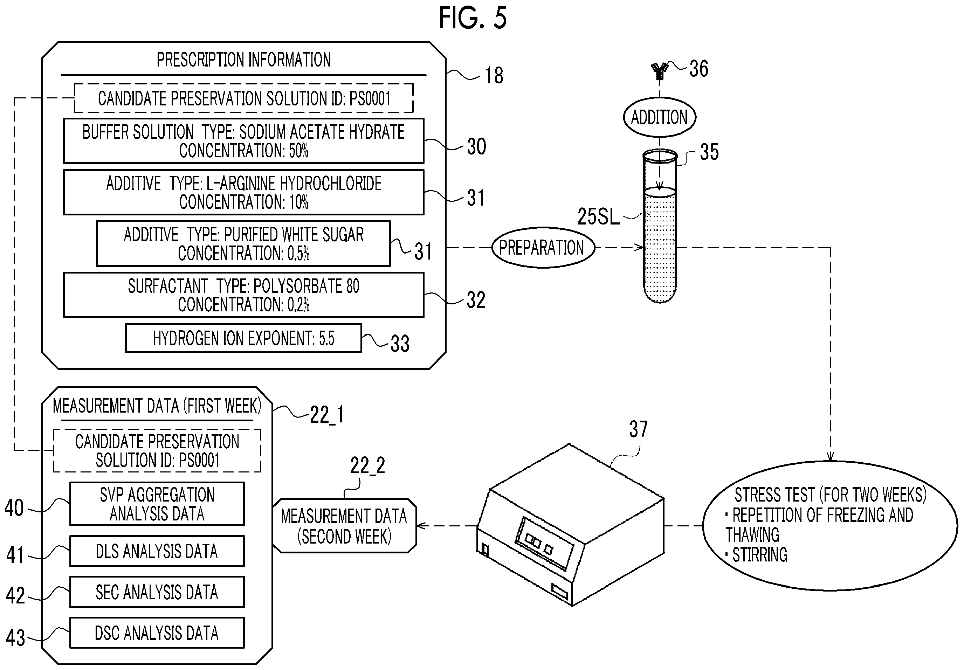

The operator terminal 11 transmits a first prediction request 15 to the pharmaceutical support server 10. The first prediction request 15 is a request for the pharmaceutical support server 10 to predict the preservation stability of a candidate preservation solution 25 (see Fig. 2) which is a candidate for a preservation solution for a biopharmaceutical. The first prediction request 15 includes antibody information 16 and a prescription information group 17. The antibody information 16 is information related to an antibody 36 (see Fig. 5) which is an active ingredient of the biopharmaceutical. The operator operates the input device 14 to input the antibody information 16. The antibody 36 is an example of a "protein" according to the technology of the present disclosure, and the antibody information 16 is an example of "protein information" according to the technology of the present disclosure.

The prescription information group 17 includes a plurality of prescription information items 18. The prescription information 18 is registered for each of a plurality of types, for example, several to several tens of types of candidate preservation solutions 25. The prescription information 18 is information related to the prescription of the candidate preservation solution 25. The operator also operates the input device 14 to input the prescription information 18. In addition, the first prediction request 15 also includes terminal identification data (ID) for uniquely identifying the operator terminal 11 which is a transmission source of the first prediction request 15, which is not illustrated.

In a case where the first prediction request 15 is received, the pharmaceutical support server 10 derives a first prediction result 19 of the preservation stability of the candidate preservation solution 25. The pharmaceutical support server 10 delivers the first prediction result 19 to the operator terminal 11 which is the transmission source of the first prediction request 15. In a case where the first prediction result 19 is received, the operator terminal 11 displays the first prediction result 19 on the display 13 such that the operator views the first prediction result 19.

As illustrated in Fig. 2 as an example, the operator selects the candidate preservation solution 25 predicted to have high preservation stability from the plurality of types of candidate preservation solutions 25 with reference to the first prediction result 19. Hereinafter, the selected candidate preservation solution 25 is referred to as a selected candidate preservation solution 25SL. Fig. 2 illustrates a case where a total of five types of selected candidate preservation solutions 25SL having candidate preservation solution IDs of PS0001, PS0004, PS0005, PS0008, and PS0010 are selected from 10 types of candidate preservation solutions 25 having candidate preservation solution IDs of PS0001 to PS0010.

Returning to Fig. 1, the operator terminal 11 transmits a second prediction request 20 to the pharmaceutical support server 10. The second prediction request 20 is a request for the pharmaceutical support server 10 to predict the preservation stability of the selected candidate preservation solution 25SL. The second prediction request 20 includes a measurement data group 21. The measurement data group 21 includes a plurality of measurement data items 22. The measurement data 22 is registered for each selected candidate preservation solution 25SL. The measurement data 22 is data indicating the preservation stability of the selected candidate preservation solution 25SL measured by an actual test. The operator also operates the input device 14 to input the measurement data 22. Similarly to the first prediction request 15, the second prediction request 20 also includes, for example, a terminal ID for uniquely identifying the operator terminal 11 which is a transmission source of the second prediction request 20, which is not illustrated.

In a case where the second prediction request 20 is received, the pharmaceutical support server 10 derives a second prediction result 23 of the preservation stability of the selected candidate preservation solution 25SL. The pharmaceutical support server 10 delivers the second prediction result 23 to the operator terminal 11 which is the transmission source of the second prediction request 20. In a case where the second prediction result 23 is received, the operator terminal 11 displays the second prediction result 23 on the display 13 such that the operator views the second prediction result 23.

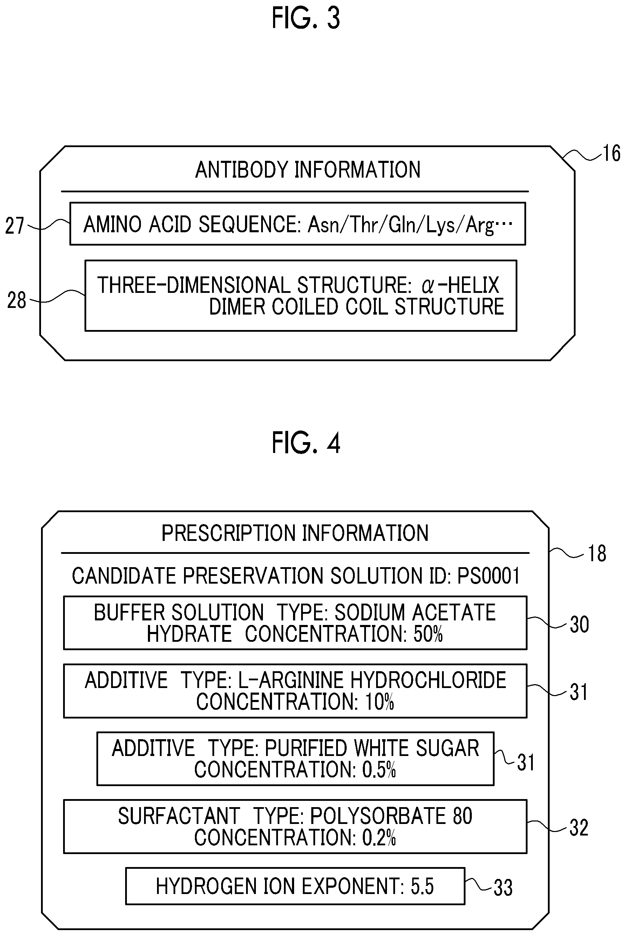

As illustrated in Fig. 3 as an example, the antibody information 16 includes an amino acid sequence 27 of the antibody 36 and a three-dimensional structure 28 of the antibody 36. The antibody information 16 is obtained by analyzing the antibody 36 with a well-known technique such as mass spectrometry, X-ray crystal structure analysis, or electron microscopy. In the amino acid sequence 27, the order of peptide bonds of amino acids constituting the antibody 36, such as asparagine (abbreviated to ASn), glutamine (abbreviated to Glu), and arginine (abbreviated to Arg), is described from an amino terminal to a carboxyl terminal. The amino acid sequence 27 is also referred to as a primary structure. The three-dimensional structure 28 indicates the secondary structure, tertiary structure, and quaternary structure of the amino acids constituting the antibody 36. Examples of the secondary structure include a β-sheet and a β-tum in addition to α-helix given as an example. The tertiary structure is, for example, a dimer coiled coil structure given as an example. Examples of the quaternary structure include a dimer, a trimer, and a tetramer. Some antibodies 36 do not have the quaternary structure (that is, some antibodies 36 are monomers). In this case, the quaternary structure is not registered as described in the example.

As illustrated in Fig. 4 as an example, the prescription information 18 includes a type and concentration 30 of a buffer solution, a type and concentration 31 of an additive, and a type and concentration 32 of a surfactant in the candidate preservation solution 25. The operator creates the prescription information 18, relying on the antibody information 16, his/her own experience, and the like. In some cases, a plurality of types of buffer solutions, additives, and surfactants are used as can be seen from L-arginine hydrochloride and purified white sugar which are the additives given as an example. In this case, a type and a concentration are registered for each of the plurality of types. Further, the prescription information 18 also includes a hydrogen ion exponent 33 of the candidate preservation solution 25. Furthermore, a molecular formula of each of the buffer solution, the additive, and the surfactant may be added to the prescription information 18.

As illustrated in Fig. 5 as an example, after selecting the selected candidate preservation solution 25SL as illustrated in Fig. 2, the operator actually prepares the selected candidate preservation solution 25SL in a test tube 35 on the basis of the prescription information 18 of the selected candidate preservation solution 25SL before transmitting the second prediction request 20. Then, the antibody 36 is added to the prepared selected candidate preservation solution 25SL. Then, a stress test including the repetition of freezing and thawing and stirring is performed for 2 weeks. During the stress test for two weeks, the measurement data 22 is actually measured by a measurement device 37. Fig. 5 illustrates an example in which measurement data 22_1 and measurement data 22_2 are measured in the first week and the second week of the stress test, respectively. The measurement device 37 transmits the measurement data 22 to the operator terminal 11.

The measurement data 22 includes aggregation analysis data (hereinafter, referred to as SVP aggregation analysis data) 40 of sub-visible particles (SVPs) of the antibody 36 in the selected candidate preservation solution 25SL and analysis data (hereinafter, referred to as DLS analysis data) 41 of the antibody 36 in the selected candidate preservation solution 25SL by a dynamic light scattering (DLS) method as illustrated in the measurement data 22_1 in the first week. The SVP aggregation analysis data 40 indicates the amount of SVPs of the antibody 36 in the selected candidate preservation solution 25SL that causes a reduction in the drug efficacy of the biopharmaceutical. Similarly, the DLS analysis data 41 indicates the amount of nanoparticles of the antibody 36 in the selected candidate preservation solution 25SL.

In addition, the measurement data 22 includes analysis data (hereinafter, referred to as SEC analysis data) 42 of the antibody 36 in the selected candidate preservation solution 25SL by size exclusion chromatography (SEC) and analysis data (hereinafter, referred to as DSC analysis data) 43 of the antibody 36 in the selected candidate preservation solution 25SL by differential scanning calorimetry (DSC). The SEC analysis data 42 indicates a molecular weight distribution of the antibody 36 in the selected candidate preservation solution 25SL. The DSC analysis data 43 indicates values related to thermophysical property values such as the glass transition point and crystallization temperature of the antibody 36 in the selected candidate preservation solution 25SL. In addition, in Fig. 5, for convenience, only one measurement device 37 is illustrated. However, in practice, various measurement devices 37 that measure these analysis data items 40 to 43 are prepared.

A common candidate preservation solution ID is given to the prescription information 18 and the measurement data 22 of one selected candidate preservation solution 25SL. The prescription information 18 and the measurement data 22 are associated with each other by the candidate preservation solution ID. Further, in Fig. 5, only the preparation of one selected candidate preservation solution 25SL, the addition of the antibody 36, the stress test, and the measurement of the measurement data 22 are illustrated. However, in practice, the preparation, the addition, the stress test, and the measurement of the measurement data 22 are performed for each of a plurality of types of selected candidate preservation solutions 25SL.

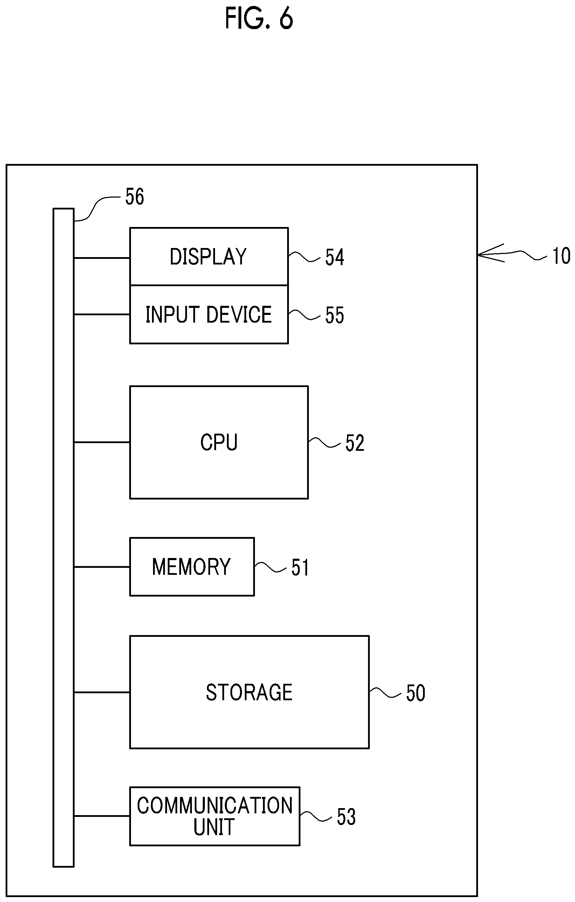

As illustrated in Fig. 6 as an example, a computer constituting the pharmaceutical support server 10 comprises a storage 50, a memory 51, a central processing unit (CPU) 52, a communication unit 53, a display 54, and an input device 55. These units are connected to one another through a bus line 56.

The storage 50 is a hard disk drive that is provided in the computer constituting the pharmaceutical support server 10 or that is connected to the computer through a cable or a network. Alternatively, the storage 50 is a disk array in which a plurality of hard disk drives are connected. The storage 50 stores, for example, a control program, such as an operating system, various application programs, and various types of data associated with these programs. In addition, a solid state drive may be used instead of the hard disk drive.

The memory 51 is a work memory used by the CPU 52 to perform processes. The CPU 52 loads the program stored in the storage 50 to the memory 51 and performs a process corresponding to the program. Therefore, the CPU 52 controls the overall operation of each unit of the computer. The CPU 52 is an example of a "processor" according to the technology of the present disclosure. In addition, the memory 51 may be provided in the CPU 52.

The communication unit 53 controls the transmission of various types of information to an external device such as the operator terminal 11. The display 54 displays various screens. The various screens have operation functions by a graphical user interface (GUI). The computer constituting the pharmaceutical support server 10 receives an input of an operation instruction from the input device 55 through the various screens. The input device 55 is, for example, a keyboard, a mouse, a touch panel, and a microphone for voice input.

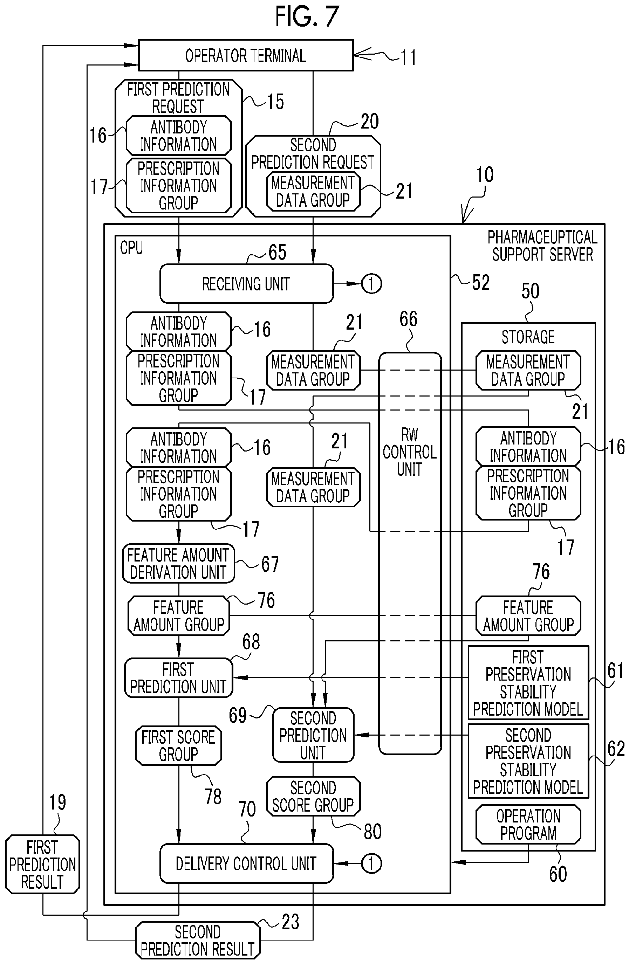

As illustrated in Fig. 7 as an example, an operation program 60 is stored in the storage 50 of the pharmaceutical support server 10. The operation program 60 is an application program for causing the computer to function as the pharmaceutical support server 10. That is, the operation program 60 is an example of "a program for operating a pharmaceutical support device" according to the technology of the present disclosure. The storage 50 also stores the antibody information 16, the prescription information group 17, the measurement data group 21, and the like. In addition, a first preservation stability prediction model 61 and a second preservation stability prediction model 62 are also stored in the storage 50. The first preservation stability prediction model 61 is an example of a "first machine learning model" according to the technology of the present disclosure. Further, the second preservation stability prediction model 62 is an example of a "second machine learning model" according to the technology of the present disclosure.

In a case where the operation program 60 is started, the CPU 52 of the computer constituting the pharmaceutical support server 10 functions as a receiving unit 65, a read and write (hereinafter, abbreviated to RW) control unit 66, a feature amount derivation unit 67, a first prediction unit 68, a second prediction unit 69, and a delivery control unit 70 in cooperation with the memory 51 and the like.

The receiving unit 65 receives the first prediction request 15 from the operator terminal 11. As described above, the first prediction request 15 includes the antibody information 16 and the prescription information group 17, and the prescription information group 17 includes the prescription information 18. Therefore, the receiving unit 65 receives the first prediction request 15 to acquire the antibody information 16 and the prescription information 18. The receiving unit 65 outputs the antibody information 16 and the prescription information group 17 to the RW control unit 66. In addition, the receiving unit 65 outputs the terminal ID of the operator terminal 11 (not illustrated) to the delivery control unit 70.

In addition, the receiving unit 65 receives the second prediction request 20 from the operator terminal 11. As described above, the second prediction request 20 includes the measurement data group 21, and the measurement data group 21 includes the measurement data 22. Therefore, the receiving unit 65 receives the second prediction request 20 to acquire the measurement data 22. The receiving unit 65 outputs the measurement data group 21 to the RW control unit 66. Further, similarly to the case where the first prediction request 15 is received, the receiving unit 65 outputs the terminal ID of the operator terminal 11 (not illustrated) to the delivery control unit 70.

The RW control unit 66 controls the storage of various types of data in the storage 50 and the reading-out of various types of data in the storage 50. For example, the RW control unit 66 stores the antibody information 16 and the prescription information group 17 from the receiving unit 65 in the storage 50. Further, the RW control unit 66 reads out the antibody information 16 and the prescription information group 17 from the storage 50 and outputs the antibody information 16 and the prescription information group 17 to the feature amount derivation unit 67. Furthermore, the RW control unit 66 reads out the first preservation stability prediction model 61 from the storage 50 and outputs the first preservation stability prediction model 61 to the first prediction unit 68.

The RW control unit 66 stores the measurement data group 21 from the receiving unit 65 in the storage 50. Further, the RW control unit 66 reads out the measurement data group 21 from the storage 50 and outputs the measurement data group 21 to the second prediction unit 69. Furthermore, the RW control unit 66 reads out the second preservation stability prediction model 62 from the storage 50 and outputs the second preservation stability prediction model 62 to the second prediction unit 69.

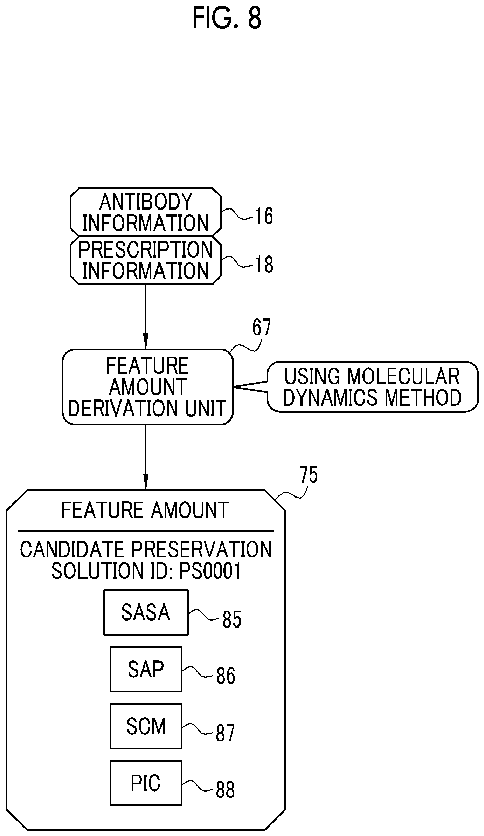

The feature amount derivation unit 67 derives a plurality of feature amounts 75 (refer to Fig. 8) respectively related to the preservation stability of the plurality of types of candidate preservation solutions 25 on the basis of the antibody information 16 and the prescription information group 17. The feature amount derivation unit 67 outputs a feature amount group 76 composed of the plurality of derived feature amounts 75 to the RW control unit 66 and the first prediction unit 68. The RW control unit 66 stores the feature amount group 76 from the feature amount derivation unit 67 in the storage 50. Further, the RW control unit 66 reads out the feature amount group 76 from the storage 50 and outputs the feature amount 75 of the selected candidate preservation solution 25SL in the feature amount group 76 to the second prediction unit 69.

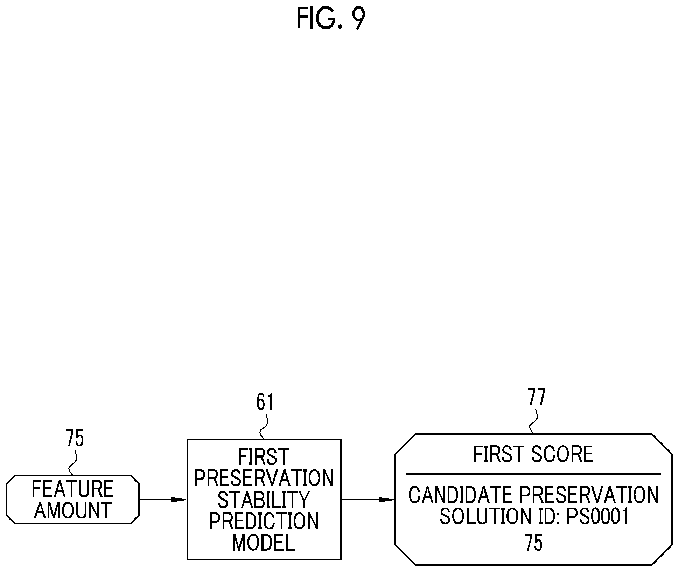

The first prediction unit 68 derives a plurality of first scores 77 (see Fig. 9) indicating the preservation stability of the plurality of types of candidate preservation solutions 25, respectively, from the feature amount group 76 using the first preservation stability prediction model 61. The first prediction unit 68 outputs a first score group 78 composed of the plurality of derived first scores 77 to the delivery control unit 70.

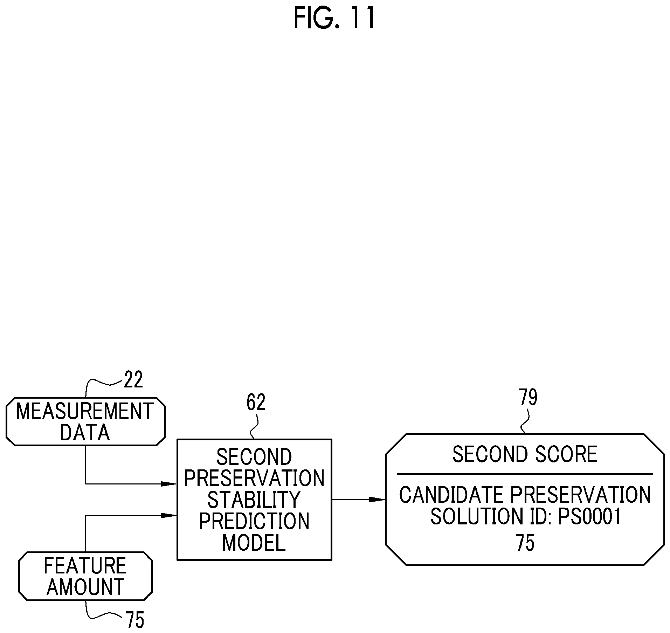

The second prediction unit 69 derives a plurality of second scores 79 (see Fig. 11) indicating the preservation stability of the plurality of types of selected candidate preservation solutions 25 SL, respectively, from the measurement data group 21 and the feature amount group 76, using the second preservation stability prediction model 62. The second prediction unit 69 outputs a second score group 80 composed of the plurality of derived second scores 79 to the delivery control unit 70.

The delivery control unit 70 generates the first prediction result 19 on the basis of the first score group 78. The delivery control unit 70 controls the delivery of the first prediction result 19 to the operator terminal 11 which is the transmission source of the first prediction request 15. In this case, the delivery control unit 70 specifies the operator terminal 11, which is the transmission source of the first prediction request 15, on the basis of the terminal ID from the receiving unit 65.

Further, the delivery control unit 70 generates the second prediction result 23 on the basis of the second score group 80. The delivery control unit 70 controls the delivery of the second prediction result 23 to the operator terminal 11 which is the transmission source of the second prediction request 20. In this case, the delivery control unit 70 specifies the operator terminal 11, which is the transmission source of the second prediction request 20, on the basis of the terminal ID from the receiving unit 65, as in the case where the first prediction result 19 is delivered.

As illustrated in Fig. 8 as an example, the feature amount derivation unit 67 derives the feature amount 75 using a molecular dynamics method. The feature amount 75 includes a hydrophobic solvent accessible surface area (hereinafter, abbreviated to SASA) 85, a spatial aggregation propensity (hereinafter, abbreviated to SAP) 86, and a spatial charge map (hereinafter, abbreviated to SCM) 87 of the antibody 36. As the SASA 85 is larger and the SAP 86 is larger, the stability of the antibody 36 is lower. The SCM 87 indicates the degree of charge of the antibody 36 for each region of the antibody 36. The SASA 85, the SAP 86, and the SCM 87 are derived on the basis of only the antibody information 16 without referring to the prescription information 18. Therefore, the SASA 85, the SAP 86, and the SCM 87 are common to each candidate preservation solution 25.

Further, the feature amount 75 also includes a preferential interaction coefficient (hereinafter, abbreviated to PIC) 88 described in Document 1. The PIC 88 indicates, for each region of the antibody 36, the ease of the binding between the antibody 36 and the additive, more specifically, the degree to which the surface of the antibody 36 is covered by the additive. Therefore, the PIC 88 is an indicator showing the compatibility between the antibody 36 and the additive. The PIC 88 makes it possible to know the possibility that the aggregation of the antibody 36 causing a reduction in the drug efficacy of the biopharmaceutical will occur. The PIC 88 is derived on the basis of both the antibody information 16 and the prescription information 18 unlike the SASA 85, the SAP 86, and the SCM 87. Therefore, the PIC 88 is different in each candidate preservation solution 25. Therefore, the feature amount group 76 is a group of a plurality of feature amounts 75 that have the SASA 85, the SAP 86, and the SCM 87 in common and have different PICs 88. The PIC 88 may be derived using a machine learning model, such as a support vector machine (SVM), instead of the molecular dynamics method. In addition, similarly to the prescription information 18 and the measurement data 22, the feature amount 75 is given the candidate preservation solution ID.

As illustrated in Fig. 9 as an example, the first prediction unit 68 inputs the feature amount 75 to the first preservation stability prediction model 61. Then, the first score 77 is output from the first preservation stability prediction model 61. The first score 77 is represented by, for example, a continuous numerical value of 0 points to 100 points. The first preservation stability prediction model 61 is a machine learning model using, for example, a support vector regression (SVR) algorithm that predicts the first score 77 which is a continuous numerical value. In addition, similarly to the feature amount 75 and the like, the first score 77 is given the candidate preservation solution ID.

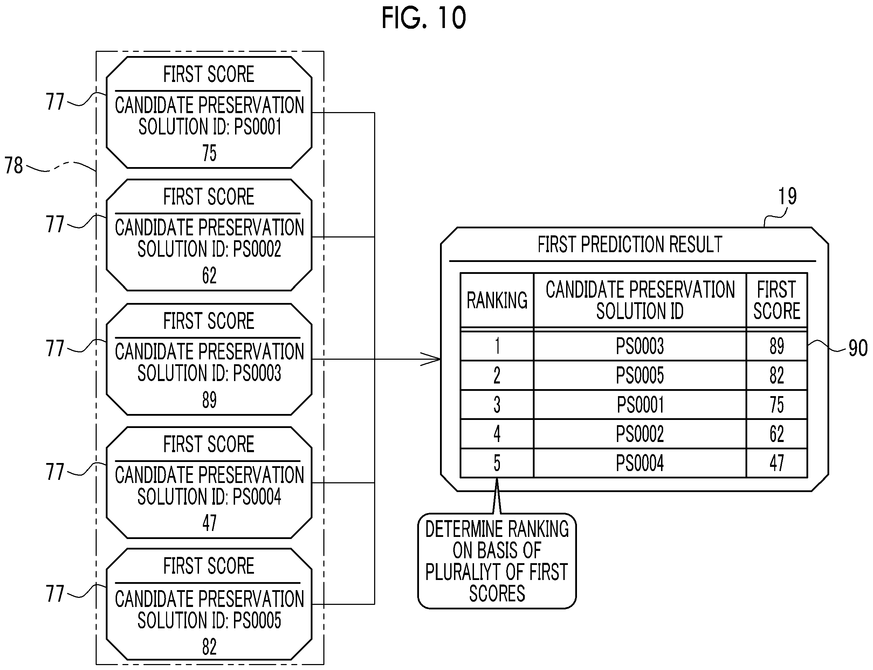

As illustrated in Fig. 10 as an example, the first prediction unit 68 directs the preservation stability prediction model 61 to output a plurality of first scores 77 for a plurality of types of candidate preservation solutions 25. The delivery control unit 70 determines ranking on the basis of the plurality of first scores 77 of the plurality of types of candidate preservation solutions 25 as illustrated in a table 90. The delivery control unit 70 delivers a determination result of the ranking illustrated in the table 90 as the first prediction result 19 to the operator terminal 11.

Fig. 10 illustrates an example in which the ranking of five types of candidate preservation solutions 25 having candidate preservation solution IDs of PS0001 to PS0005 is determined. The first scores 77 of PS0001, PS0002, PS0003, PS0004, and PS0005 are 75 points, 62 points, 89 points, 47 points, and 82 points, respectively. For the ranking, PS0003 with the highest score of 89 points is in first place, PS0005 with the second highest score of 82 points is in second place, PS0001 with 75 points is in third place, PS0002 with 62 points is in fourth place, and PS0004 with the lowest score of 47 points is in fifth place.

As illustrated in Fig. 11 as an example, the second prediction unit 69 inputs the measurement data 22 and the feature amount 75 to the second preservation stability prediction model 62. Then, the second score 79 is output from the second preservation stability prediction model 62. Here, the feature amount 75 to be input to the second preservation stability prediction model 62 is the feature amount 75 of the selected candidate preservation solution 25SL among the plurality of feature amounts 75 previously derived by the feature amount derivation unit 67. The second score 79 is represented by, for example, a continuous numerical value of 0 points to 100 points, similarly to the first score 77. The second preservation stability prediction model 62 is a machine learning model using, for example, a support vector regression (SVR) algorithm that predicts the second score 79 which is a continuous numerical value, similarly to the first preservation stability prediction model 61. In addition, similarly to the first score 77 and the like, the second score 79 is given the candidate preservation solution ID.

The first preservation stability prediction model 61 and the second preservation stability prediction model 62 may be trained in the pharmaceutical support server 10 or may be trained by a device other than the pharmaceutical support server 10. In addition, the first preservation stability prediction model 61 and the second preservation stability prediction model 62 may be continuously trained even after the operation.

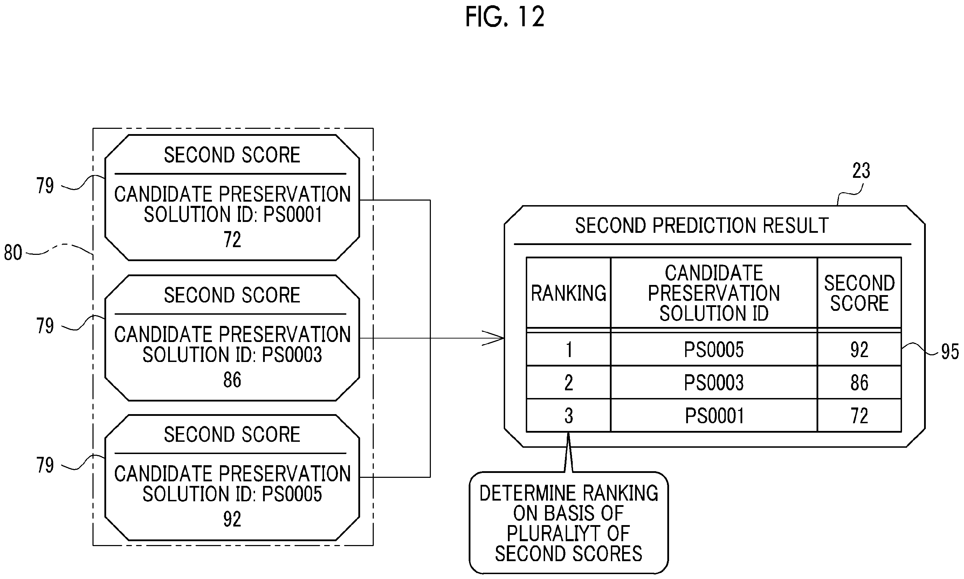

As illustrated in Fig. 12 as an example, the second prediction unit 69 directs the second preservation stability prediction model 62 to output a plurality of second scores 79 for a plurality of types of selected candidate preservation solutions 25 SL. The delivery control unit 70 determines ranking on the basis of the plurality of second scores 79 of the plurality of types of selected candidate preservation solutions 25SL as illustrated in a table 95. The delivery control unit 70 delivers a determination result of the ranking illustrated in the table 95 as the second prediction result 23 to the operator terminal 11.

Fig. 12 illustrates an example in which the ranking of three types of selected candidate preservation solutions 25SL having candidate preservation solution IDs of PS0001, PS0003, and PS0005 is determined. The second scores 79 of PS0001, PS0003, and PS0005 are 72 points, 86 points, and 92 points, respectively. For the ranking, PS0005 with the highest score of 92 points is in first place, PS0003 with the second highest score of 86 points is in second place, and PS0001 with the lowest score of 72 points is in third place.

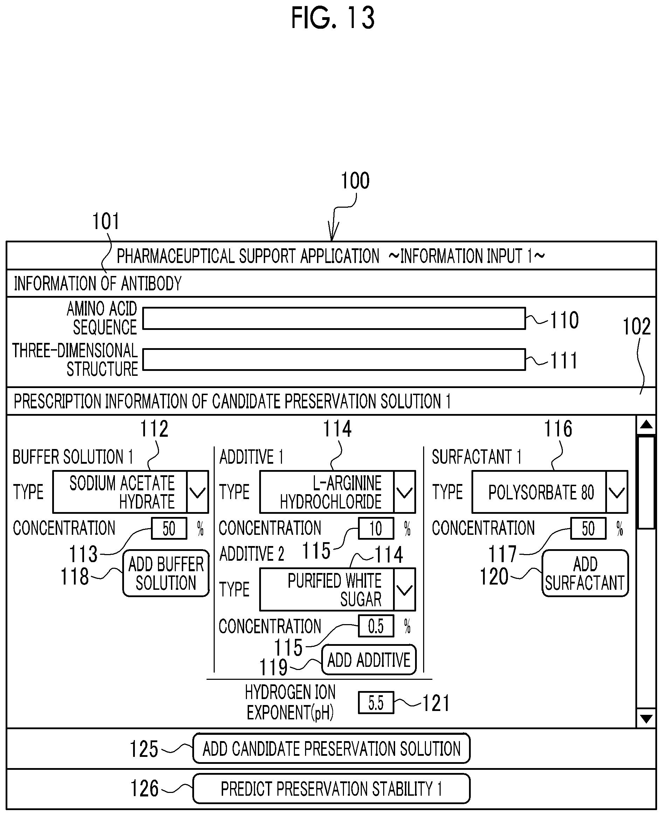

Fig. 13 illustrates an example of a first information input screen 100 that is displayed on the display 13 of the operator terminal 11. The first information input screen 100 has an input region 101 for the antibody information 16 and an input region 102 for the prescription information 18 of the candidate preservation solution 25.

An input box 110 for the amino acid sequence 27 and an input box 111 for the three-dimensional structure 28 are provided in the input region 101 for the antibody information 16. The input region 102 for the prescription information 18 is provided with a pull-down menu 112 for selecting the type of the buffer solution, an input box 113 for the concentration of the buffer solution, a pull-down menu 114 for selecting the type of the additive, an input box 115 for the concentration of the additive, a pull-down menu 116 for selecting the type of the surfactant, and an input box 117 for the concentration of the surfactant.

An add button 118 for the buffer solution is provided below the pull-down menu 112 and the input box 113 for the buffer solution. In a case where the add button 118 is selected, the pull-down menu 112 and the input box 113 for the buffer solution are added. An add button 119 for the additive is provided below the pull-down menu 114 and the input box 115 for the additive. In a case where the add button 119 is selected, the pull-down menu 114 and the input box 115 for the additive are added. An add button 120 for the surfactant is provided below the pull-down menu 116 and the input box 117 for the surfactant. In a case where the add button 120 is selected, the pull-down menu 116 and the input box 117 for the surfactant are added. Further, an input box 121 for the hydrogen ion exponent is provided in a lower portion of the input region 102.

An add button 125 for the candidate preservation solution 25 is provided in the lower portion of the input region 102 for the prescription information 18. In a case where the add button 125 is selected, the input region 102 for the prescription information 18 is added.

A first prediction button 126 is provided below the add button 125 for the candidate preservation solution 25. In a case where the first prediction button 126 is selected, the first prediction request 15 including the antibody information 16 and the prescription information group 17 is transmitted from the operator terminal 11 to the pharmaceutical support server 10. The antibody information 16 includes contents that have been input to the input boxes 110 and 111. The prescription information group 17 is composed of contents selected in the pull-down menus 112, 114, and 116 and contents input to the input boxes 113, 115, 117, and 121.

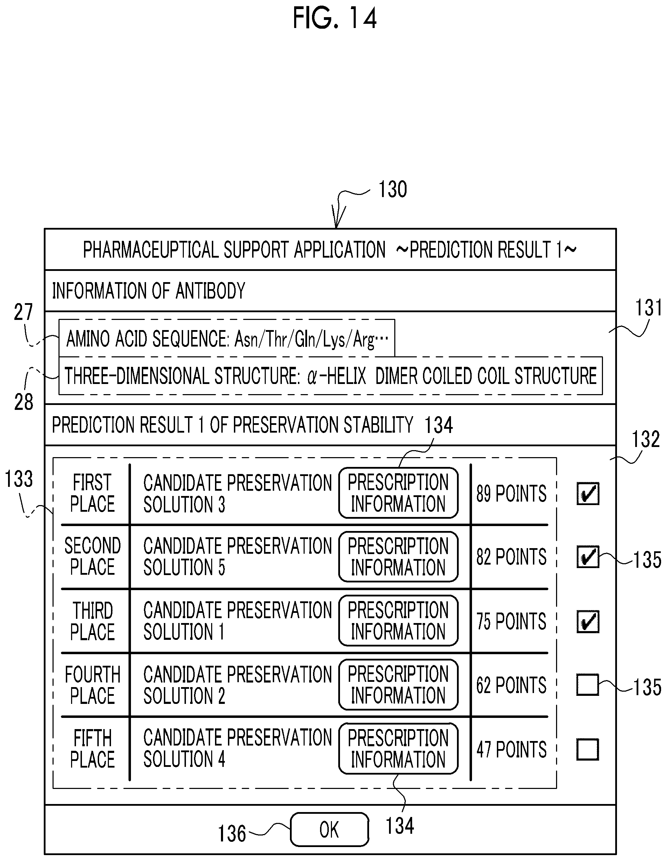

Fig. 14 illustrates an example of a first prediction result display screen 130 that is displayed on the display 13 of the operator terminal 11 in a case where the first prediction result 19 is received from the pharmaceutical support server 10. The first prediction result display screen 130 has a display region 131 for the antibody information 16 and a display region 132 for the first prediction result 19. The amino acid sequence 27 and the three-dimensional structure 28 are displayed in the display region 131 for the antibody information 16. A table 133 showing the ranking and the first score 77 of each candidate preservation solution 25 is displayed in the display region 132 for the first prediction result 19. A display button 134 for the prescription information 18 is provided in the table 133. In a case where the display button 134 is selected, a display screen for the prescription information 18 of the candidate preservation solution 25 is displayed on the first prediction result display screen 130 in a pop-up manner.

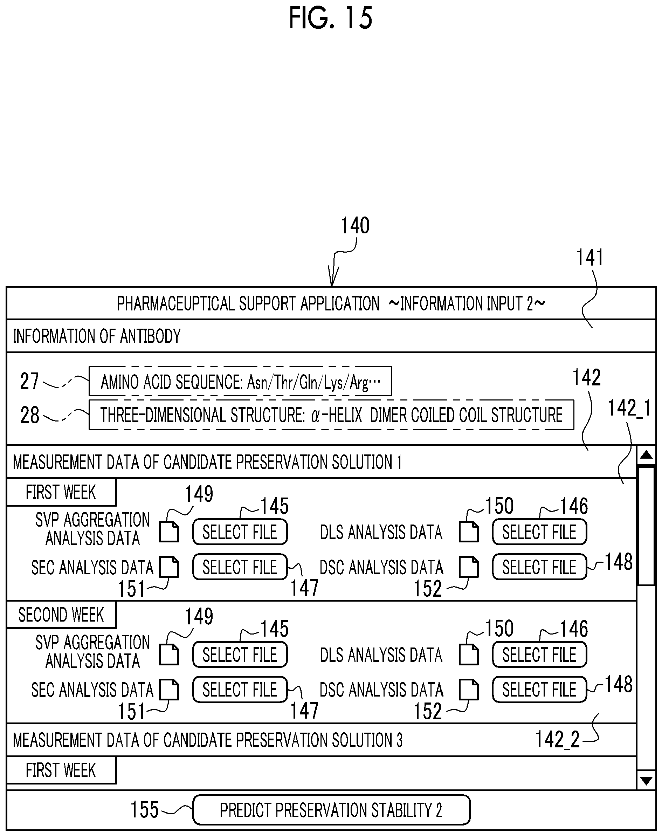

A check box 135 is provided beside each candidate preservation solution 25 in the table 133. A check box 135 is checked by the operator in a case where the operator wants to use the candidate preservation solution 25 as the selected candidate preservation solution 25SL. In a case where an OK button 136 is selected in a state in which the check box 135 is checked, the candidate preservation solution ID of the candidate preservation solution 25 whose check box 135 has been checked is stored as the candidate preservation solution ID of the selected candidate preservation solution 25SL in the storage 50 of the pharmaceutical support server 10. The candidate preservation solution ID stored in this way is used, for example, in a case where a second information input screen 140 (see Fig. 15) is generated or in a case where the feature amount 75 of the selected candidate preservation solution 25SL is specified.

Fig. 15 illustrates an example of the second information input screen 140 that is displayed on the display 13 of the operator terminal 11. The second information input screen 140 has a display region 141 for the amino acid sequence 27 and the three-dimensional structure 28 of the antibody information 16 and an input region 142 for the measurement data 22 of the selected candidate preservation solution 25SL. The input region 142 for the measurement data 22 is divided into an input region 142_1 for the measurement data 22_1 in the first week and an input region 142_2 for the measurement data 22_2 in the second week.

The input region 142 for the measurement data 22 is provided with a file selection button 145 for selecting a file of the SVP aggregation analysis data 40 and a file selection button 146 for selecting a file of the DLS analysis data 41. In addition, the input region 142 for the measurement data 22 is provided with a file selection button 147 for selecting a file of the SEC analysis data 42 and a file selection button 148 for selecting a file of the DSC analysis data 43.

In a case where the file of each of the analysis data items 40 to 43 is selected, file icons 149, 150, 151, and 152 are displayed beside the file selection buttons 145 to 148, respectively. The file icons 149 to 152 are not displayed in a case where no files are selected.

A second prediction button 155 is provided in a lower portion of the input region 142 for the measurement data 22. In a case where the second prediction button 155 is selected, the second prediction request 20 including the measurement data group 21 is transmitted from the operator terminal 11 to the pharmaceutical support server 10. The measurement data group 21 is composed of the analysis data items 40 to 43 selected by the file selection buttons 145 to 148, respectively.

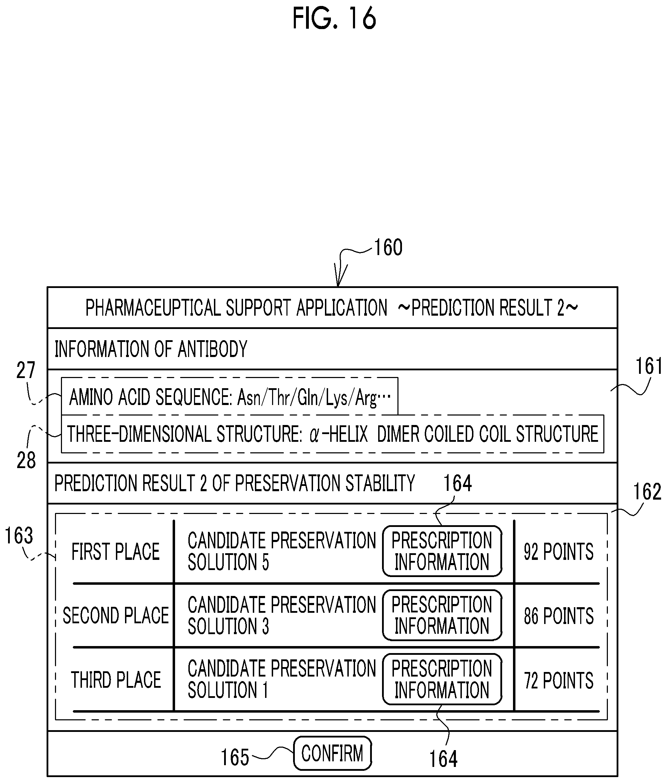

Fig. 16 illustrates an example of a second prediction result display screen 160 that is displayed on the display 13 of the operator terminal 11 in a case where the second prediction result 23 is received from the pharmaceutical support server 10. The second prediction result display screen 160 has a display region 161 for the amino acid sequence 27 and the three-dimensional structure 28 of the antibody information 16 and a display region 162 for the second prediction result 23. A table 163 showing the ranking and the second score 79 of each selected candidate preservation solution 25SL is displayed in the display region 162 for the second prediction result 23. A display button 164 for the prescription information 18 is provided in the table 163. In a case where the display button 164 is selected, a display screen for the prescription information 18 of the selected candidate preservation solution 25SL is displayed on the second prediction result display screen 160 in a pop-up manner. The second prediction result display screen 160 is removed by the selection of a confirm button 165.

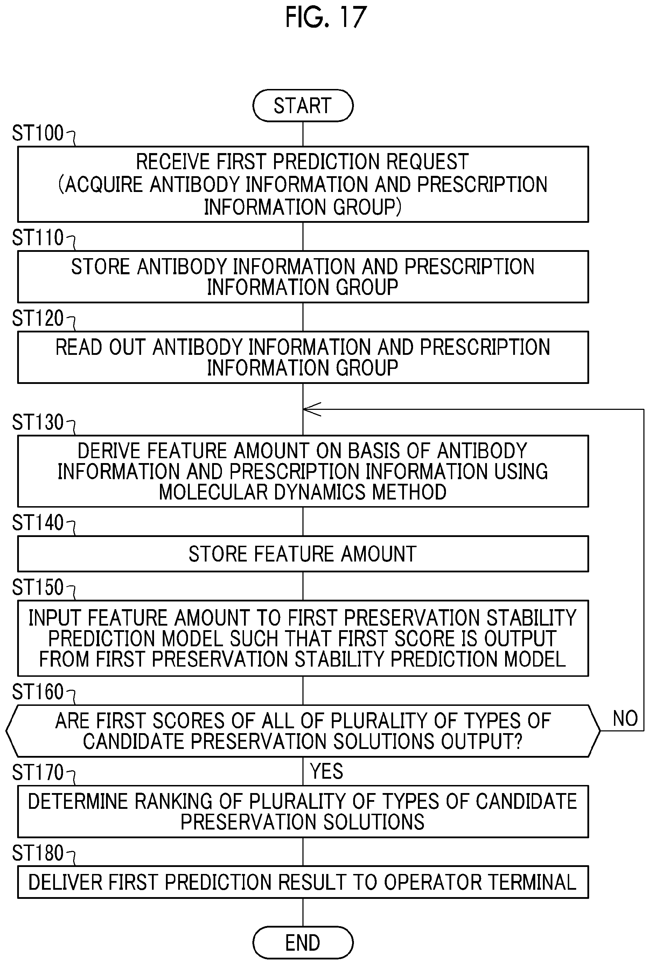

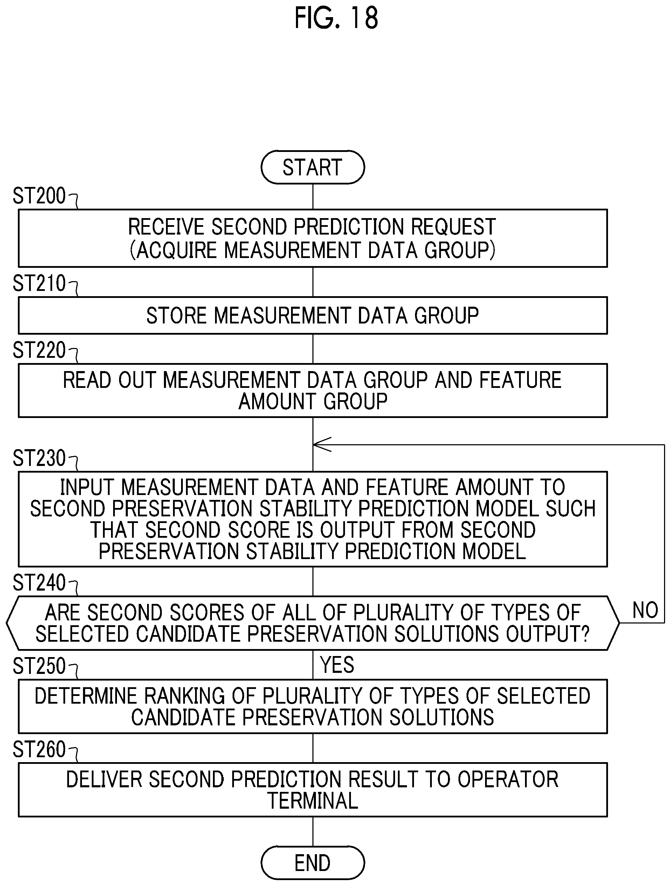

Next, the operation of the above-described configuration will be described with reference to flowcharts illustrated in Figs. 17 and 18. First, in a case where the operation program 60 is started in the pharmaceutical support server 10, the CPU 52 of the pharmaceutical support server 10 functions as the receiving unit 65, the RW control unit 66, the feature amount derivation unit 67, the first prediction unit 68, the second prediction unit 69, and the delivery control unit 70 as illustrated in Fig. 7.

The first information input screen 100 illustrated in Fig. 13 is displayed on the display 13 of the operator terminal 11. The operator inputs the desired antibody information 16 and prescription information group 17 to the first information input screen 100 and then selects the first prediction button 126. Then, the first prediction request 15 is transmitted from the operator terminal 11 to the pharmaceutical support server 10.

In the pharmaceutical support server 10, the first prediction request 15 is received by the receiving unit 65. Therefore, the antibody information 16 and the prescription information group 17 (a plurality of prescription information items 18) are acquired (Step ST100). The antibody information 16 and the prescription information group 17 are output from the receiving unit 65 to the RW control unit 66 and are stored in the storage 50 under the control of the RW control unit 66 (Step ST110).

The RW control unit 66 reads out the antibody information 16 and the prescription information group 17 from the storage 50 (Step ST120). The antibody information 16 and the prescription information group 17 are output from the RW control unit 66 to the feature amount derivation unit 67.

As illustrated in Fig. 8, the feature amount derivation unit 67 derives the feature amount 75 on the basis of the antibody information 16 and the prescription information 18 using the molecular dynamics method (Step ST130). The feature amount 75 is output from the feature amount derivation unit 67 to the RW control unit 66 and is stored in the storage 50 under the control of the RW control unit 66 (Step ST140). In addition, the feature amount 75 is output from the feature amount derivation unit 67 to the first prediction unit 68.

Then, as illustrated in Fig. 9, in the first prediction unit 68, the feature amount 75 is input to the first preservation stability prediction model 61, and the first score 77 is output from the first preservation stability prediction model 61 (Step ST150). The first score 77 is output from the first prediction unit 68 to the delivery control unit 70. The processes in Steps ST130 to ST150 are repeatedly continued while the first scores 77 of all of the plurality of types of candidate preservation solutions 25 are not output (NO in Step ST160).

In a case where the first scores 77 of all of the plurality of types of candidate preservation solutions 25 are output (YES in Step ST160), the delivery control unit 70 determines the ranking of the candidate preservation solutions 25 on the basis of a plurality of first scores 77 of the plurality of types of candidate preservation solutions 25 as illustrated in Fig. 10 (Step ST170). The determination result of the ranking is delivered as the first prediction result 19 to the operator terminal 11, which is the transmission source of the first prediction request 15, under the control of the delivery control unit 70 (Step ST180).

The first prediction result display screen 130 illustrated in Fig. 14 is displayed on the display 13 of the operator terminal 11. The operator views the first prediction result display screen 130, checks the check box 135 of the candidate preservation solution 25 to be used as the selected candidate preservation solution 25SL, and selects the OK button 136. The RW control unit 66 stores the candidate preservation solution ID of the candidate preservation solution 25 whose check box 135 has been checked as the candidate preservation solution ID of the selected candidate preservation solution 25SL.

Then, as illustrated in Fig. 5, the operator actually performs the preparation of the selected candidate preservation solution 25SL, the addition of the antibody 36, the stress test, and the measurement of the measurement data 22.

In a case where the measurement data 22 is transmitted, the operator operates the operator terminal 11 to display the second information input screen 140 illustrated in Fig. 15 on the display 13. The operator inputs the desired measurement data 22 to the second information input screen 140 and then selects the second prediction button 155. Then, the second prediction request 20 is transmitted from the operator terminal 11 to the pharmaceutical support server 10.

In the pharmaceutical support server 10, the second prediction request 20 is received by the receiving unit 65. Therefore, the measurement data group 21 (a plurality of measurement data items 22) is acquired (Step ST200). The measurement data group 21 is output from the receiving unit 65 to the RW control unit 66 and is stored in the storage 50 under the control of the RW control unit 66 (Step ST210).

The RW control unit 66 reads the measurement data group 21 and the feature amount 75 of the selected candidate preservation solution 25SL from the storage 50 (Step ST220). The measurement data group 21 and the feature amount 75 are output from the RW control unit 66 to the second prediction unit 69.

Then, as illustrated in Fig. 11, in the second prediction unit 69, the measurement data 22 and the feature amount 75 are input to the second preservation stability prediction model 62, and the second score 79 is output from the second preservation stability prediction model 62 (Step ST230). The second score 79 is output from the second prediction unit 69 to the delivery control unit 70. The process in Step ST230 is repeatedly continued while the second scores 79 of all of the plurality of types of selected candidate preservation solutions 25SL are not output (NO in Step ST240).

In a case where the second scores 79 of all of the plurality of types of selected candidate preservation solutions 25SL are output (YES in Step ST240), the delivery control unit 70 determines the ranking of the selected candidate preservation solutions 25SL on the basis of a plurality of scores 79 of the plurality of types of selected candidate preservation solutions 25SL as illustrated in Fig. 12 (Step ST250). The determination result of the ranking is delivered as the second prediction result 23 to the operator terminal 11, which is the transmission source of the second prediction request 20, under the control of the delivery control unit 70 (Step ST260).

The second prediction result display screen 160 illustrated in Fig. 16 is displayed on the display 13 of the operator terminal 11. The operator views the second prediction result display screen 160 and selects one selected candidate preservation solution 25SL to be adopted as the preservation solution for the biopharmaceutical.

As described above, the CPU 52 of the pharmaceutical support server 10 comprises the receiving unit 65, the feature amount derivation unit 67, the first prediction unit 68, and the second prediction unit 69. The receiving unit 65 receives the first prediction request 15 to acquire the antibody information 16 related to the antibody 36 included in the biopharmaceutical to be preserved and a plurality of prescription information items 18 related to the prescription of each of a plurality of candidate preservation solutions 25 which are candidates for the preservation solution for the biopharmaceutical to be preserved. The feature amount derivation unit 67 derives a plurality of feature amounts 75 related to the preservation stability of each of a plurality of types of candidate preservation solutions 25 on the basis of the antibody information 16 and the prescription information 18. The first prediction unit 68 inputs the feature amount 75 to the first preservation stability prediction model 61 such that the first score 77 indicating the preservation stability of the candidate preservation solution 25 is output from the first preservation stability prediction model 61.

The receiving unit 65 receives the second prediction request 20 to acquire the measurement data 22 indicating the preservation stability measured by the actual test for the selected candidate preservation solution 25SL selected from a plurality of types of candidate preservation solutions 25 on the basis of the first score 77. The second prediction unit 69 inputs the measurement data 22 and the feature amount 75 of the selected candidate preservation solution 25SL among the plurality of derived feature amounts 75 to the second preservation stability prediction model 62 such that the second score 79 indicating the preservation stability of the selected candidate preservation solution 25 SL is output from the second preservation stability prediction model 62. A plurality of types of candidate preservation solutions 25 are narrowed down to the selected candidate preservation solutions 25SL whose number is smaller than the number of candidate preservation solutions 25 on the basis of the first scores 77, and the second score 79 is derived on the basis of the measurement data 22 of the selected candidate preservation solution 25SL obtained by the actual test. Therefore, it is possible to efficiently predict the preservation stability of the preservation solution with high accuracy.

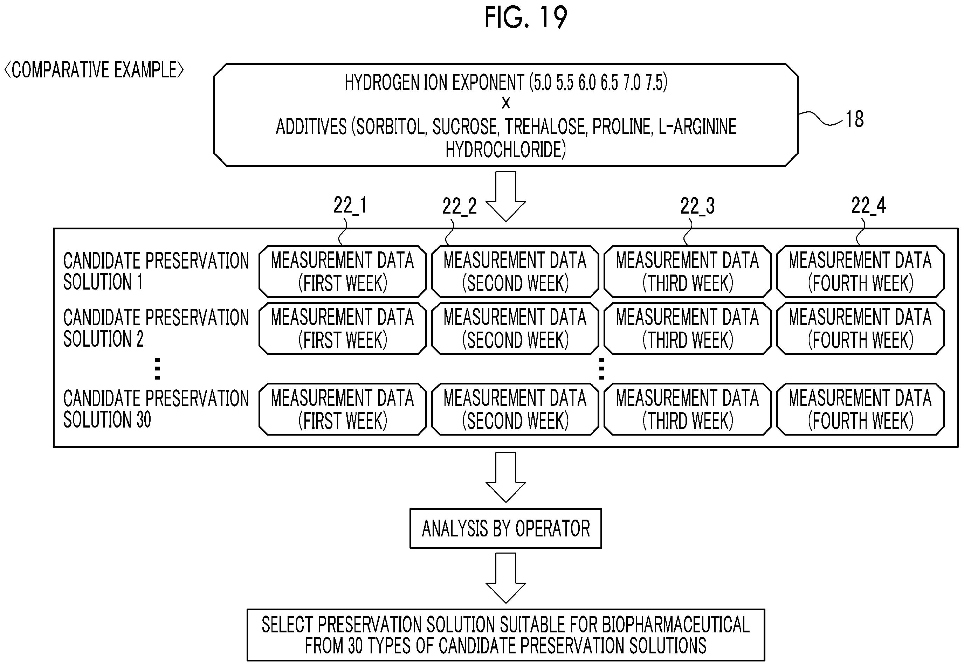

In addition, as illustrated in Figs. 19, 20, and 21 as an example, it is also possible to reduce a complicated operation of preparing a plurality of types of candidate preservation solutions 25 and checking the preservation stability of each candidate preservation solution 25 with the actual test.

Figs. 19 to 21 illustrate an example in which the hydrogen ion exponent is divided into 6 levels of 5.0, 5.5, 6.0, 6.5, 7.0, and 7.5 at an interval of 0.5, any one of five types of additives of sorbitol, sucrose, trehalose, proline, and L-arginine hydrochloride is used as the additive, and one candidate preservation solution 25 to be adopted as the preservation solution for the biopharmaceutical is selected from 30 (= 6 × 5) types of candidate preservation solutions 25.

Fig. 19 illustrates a procedure according to the related art as a comparative example. In the related art, the stress test was performed on each of 30 types of candidate preservation solutions 25 for four weeks, and measurement data 22_1 in the first week, measurement data 22_2 in the second week, measurement data 22_3 in the third week, and measurement data 22_4 in the fourth week were actually measured. The operator analyzed the preservation stability of each candidate preservation solution 25 on the basis of the measurement data 22 and selected one candidate preservation solution 25 to be adopted as the preservation solution for the biopharmaceutical from the 30 types of candidate preservation solutions 25.

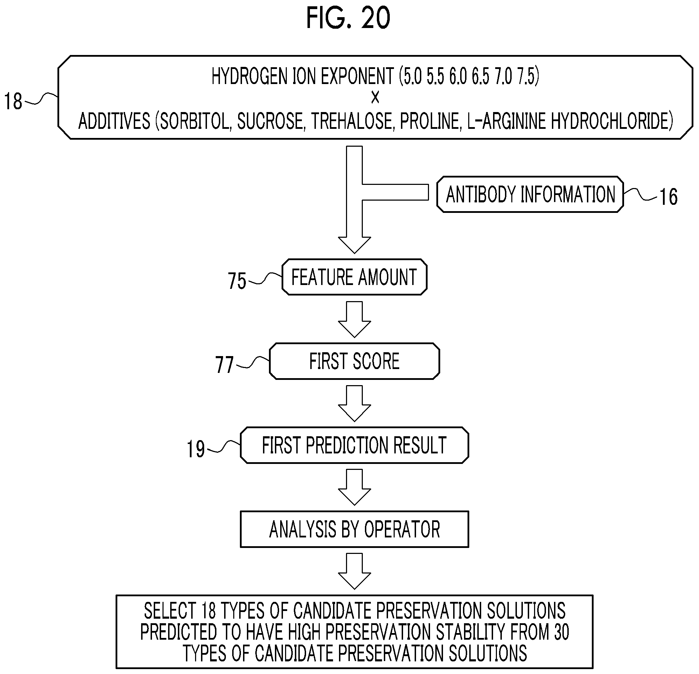

In contrast, in the procedure according to this example, first, as illustrated in Fig. 20, the feature amount 75 related to the preservation stability of the candidate preservation solution 25 is derived on the basis of the antibody information 16 and the prescription information 18. In addition, the first score 77 indicating the preservation stability of the candidate preservation solution 25 is derived on the basis of the feature amount 75. Then, the first prediction result 19 corresponding to the first score 77 is presented to the operator. The operator analyzes the preservation stability of each candidate preservation solution 25 on the basis of the first prediction result 19 and selects the candidate preservation solution 25 predicted to have high preservation stability as the selected candidate preservation solution 25SL from 30 types of candidate preservation solutions 25. Fig. 20 illustrates a case where 18 types of selected candidate preservation solutions 25SL are selected from the 30 types of candidate preservation solutions 25.

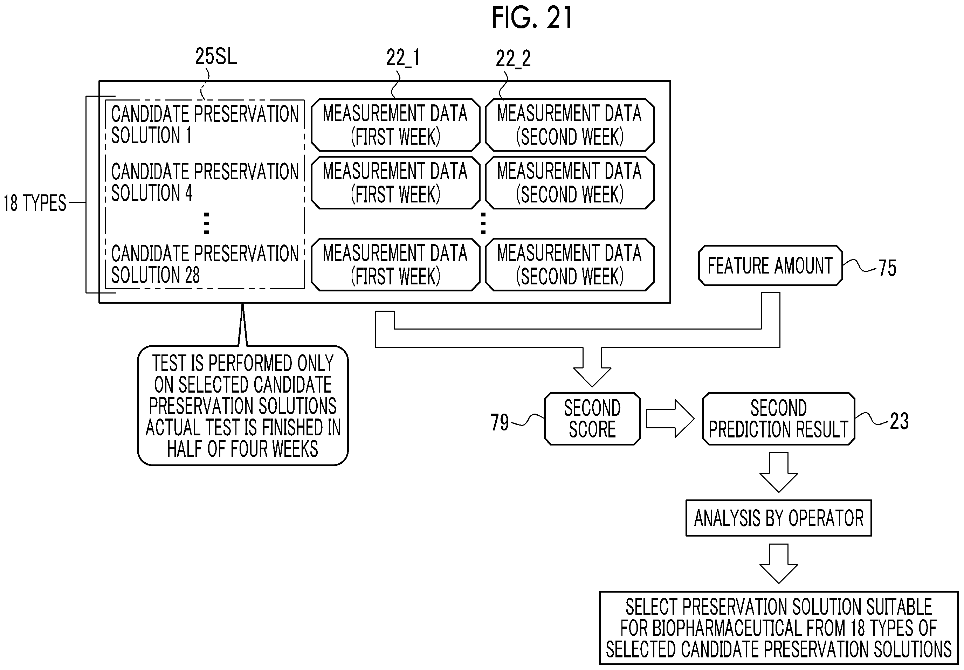

Then, as illustrated in Fig. 21, the operator finishes the stress test on the 18 types of selected candidate preservation solutions 25SL in two weeks that are half of four weeks. The second score 79 indicating the preservation stability of the selected candidate preservation solution 25SL is derived from the measurement data 22_1 in the first week and the measurement data 22_2 in the second week measured by the stress test for the two weeks and the feature amount 75 derived from the antibody information 16 and the prescription information 18. Then, the second prediction result 23 corresponding to the second score 79 is presented to the operator. The operator analyzes the preservation stability of each selected candidate preservation solution 25SL on the basis of the second prediction result 23 and selects one selected candidate preservation solution 25SL to be adopted as the preservation solution for the biopharmaceutical from the 18 types of selected candidate preservation solutions 25SL.

As described above, while it is necessary to perform the stress test on all of the 30 types of candidate preservation solutions 25 for four weeks in the procedure according to the related art, the stress test can be completed on the 18 types of selected candidate preservation solutions 25SL in two weeks in the procedure according to this example. Therefore, it is possible to reduce a complicated operation of preparing a plurality of types of candidate preservation solutions 25 and checking the preservation stability of each candidate preservation solution 25 with the actual test.

Further, as the procedure according to the related art, there is a method that determines the prescription of the preservation solution step by step by, for example, narrowing down the type and concentration of the buffer solution in a first stage, narrowing down the type and concentration of the additive in a second stage, ..., narrowing down the numerical range of the hydrogen ion exponent in an N-th stage. According to the procedure of this example, it is possible to reduce the operation in each stage. In addition, in some cases, the stage can be omitted. For example, the first stage is omitted, and the method starts from the second stage.

As illustrated in Fig. 3, the antibody information 16 includes the amino acid sequence 27 of the antibody 36 and the three-dimensional structure 28 of the antibody 36. Therefore, it is possible to derive the feature amount 75 that more accurately indicates the preservation stability of the candidate preservation solution 25. In addition, it is sufficient that the antibody information 16 includes at least one of the amino acid sequence 27 of the antibody 36 or the three-dimensional structure 28 of the antibody 36.

As illustrated in Fig. 4, the prescription information 18 includes the types and concentrations 30 to 32 of each of the buffer solution, the additive, and the surfactant included in the candidate preservation solution 25 and the hydrogen ion exponent 33 of the candidate preservation solution 25. Therefore, it is possible to derive the feature amount 75 that more accurately indicates the preservation stability of the candidate preservation solution 25.

As illustrated in Fig. 8, the feature amount derivation unit 67 derives the feature amount 75 using the molecular dynamics method. The molecular dynamics method is a computer simulation method that is popular in the field of biotechnology and can be introduced without any particular barriers. Therefore, it is possible to easily derive the feature amount 75 without requiring a lot of time and effort.

As illustrated in Fig. 8, the feature amount 75 includes the SASA 85, the SAP 86, and the SCM 87 of the antibody 36. In addition, the feature amount 75 includes the PIC 88 which is an indicator showing the compatibility between the antibody 36 and the additive included in the candidate preservation solution 25. Therefore, the first score 77 that more accurately indicates the preservation stability of the candidate preservation solution 25 (the stability of the antibody 36) can be output from the first preservation stability prediction model 61. Further, the feature amount 75 may include at least one of the SASA 85, the SAP 86, or the SCM 87 of the antibody 36.

As illustrated in Fig. 5, the measurement data 22 includes the SVP aggregation analysis data 40, the DLS analysis data 41, the SEC analysis data 42, and the DSC analysis data 43 of the antibody 36 in the candidate preservation solution 25. Therefore, the second score 79 that more accurately indicates the preservation stability of the candidate preservation solution 25 (the stability of the antibody 36) can be output from the second preservation stability prediction model 62. In addition, it is sufficient that the measurement data 22 includes at least one of the SVP aggregation analysis data 40, the DLS analysis data 41, the SEC analysis data 42, or the DSC analysis data 43.

As illustrated in Fig. 10, the first prediction unit 68 outputs a plurality of first scores 77 for a plurality of types of candidate preservation solutions 25. The delivery control unit 70 determines the ranking of the candidate preservation solutions 25 on the basis of the plurality of first scores 77, and delivers and presents the determination result of the ranking as the first prediction result 19 to the operator terminal 11. Therefore, the operator selects the selected candidate preservation solution 25SL.

As illustrated in Fig. 12, the second prediction unit 69 outputs a plurality of second scores 79 for a plurality of types of selected candidate preservation solutions 25SL. The delivery control unit 70 determines the ranking of the selected candidate preservation solutions 25SL on the basis of the plurality of second scores 79, and delivers and presents the determination result of the ranking as the second prediction result 23 to the operator terminal 11. Therefore, the operator selects the selected candidate preservation solution 25SL to be adopted as the preservation solution for the biopharmaceutical.

The biopharmaceutical including the antibody 36 as a protein is called an antibody drug and is widely used not only for the treatment of chronic diseases, such as cancer, diabetes, and rheumatoid arthritis, but also for the treatment of rare diseases such as hemophilia and a Crohn's disease. Therefore, according to this example in which the protein is the antibody 36, it is possible to further promote the development of antibody drugs widely used for the treatment of various diseases.

### [Second Embodiment]

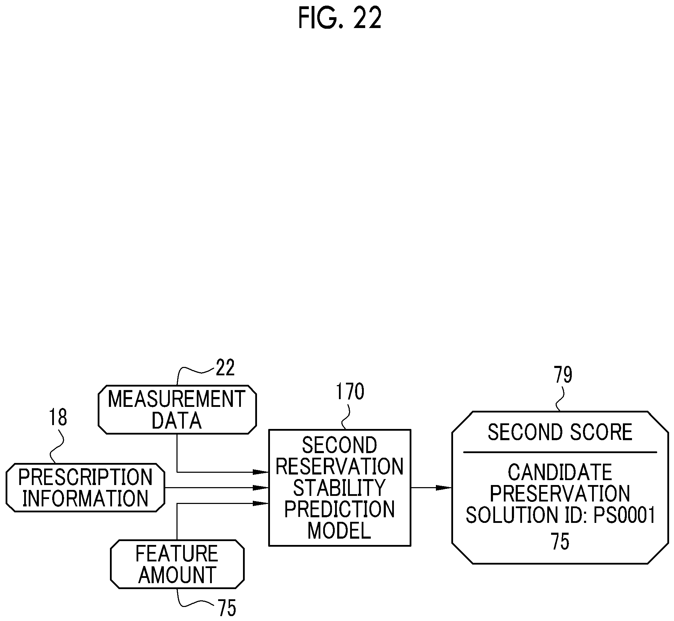

In a second embodiment illustrated in Fig. 22 as an example, in addition to the measurement data 22 and the feature amount 75, the prescription information 18 of the selected candidate preservation solution 25SL is input to the second preservation stability prediction model 170. For the concentrations of the buffer solution, the additive, and the surfactant, the numerical values of the concentrations are input without any change. Meanwhile, for the types of the buffer solution, the additive, and the surfactant, numerical values set in advance for each type are input. Since the prescription information 18 is added to the input data of the second preservation stability prediction model 170, it is possible to further improve the prediction accuracy of the second score 79. Further, the antibody information 16 may be further input. Even in this case, for the amino acid sequence 27 and the three-dimensional structure 28, preset numerical values are input.

In each of the above-described embodiments, the description has been made on the premise that there are a plurality of types of selected candidate preservation solutions 25SL. However, the present disclosure is not limited thereto, and one type of selected candidate preservation solution 25SL may be provided. Further, the example has been described in which the operator checks the check box 135 to select the selected candidate preservation solution 25SL. However, the present disclosure is not limited thereto. The pharmaceutical support server 10 may automatically select a predetermined number of candidate preservation solutions 25 having the first scores 77 equal to or greater than the threshold value as the selected candidate preservation solutions 25SL without the help of the operator.

The protein is not limited to the antibody 36 given as an example. Examples of the protein include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (a seventh factor, an eighth factor, a ninth factor, or the like), an enzyme (a lysosomal enzyme, a deoxyribonucleic acid (DNA) degrading enzyme, or the like), a fragment crystallizable (Fc) fusion protein, a receptor, albumin, and a protein vaccine. In addition, examples of the antibody include a bispecific antibody, an antibody-drug conjugate, a low-molecular-weight antibody, and a sugar-chain-modified antibody.

Instead of delivering the first prediction result 19 and the second prediction result 23 from the pharmaceutical support server 10 to the operator terminal 11, screen data of the first prediction result display screen 130 illustrated in Fig. 14 and the second prediction result display screen 160 illustrated in Fig. 16 may be delivered from the pharmaceutical support server 10 to the operator terminal 11.

The aspect in which the first prediction result 19 and the second prediction result 23 are provided to be viewed by the operator is not limited to the first prediction result display screen 130 and the second prediction result display screen 160. A printed matter of the first prediction result 19 and the second prediction result 23 may be provided to the operator, or an e-mail to which the first prediction result 19 and the second prediction result 23 are attached may be transmitted to a portable terminal of the operator.

The pharmaceutical support server 10 may be installed in each pharmaceutical facility or may be installed in a data center independent of the pharmaceutical facility. In addition, the operator terminal 11 may be configured to have some or all of the functions of each of the processing units 65 to 69 of the pharmaceutical support server 10.

The hardware configuration of the computer constituting the pharmaceutical support server 10 according to the technology of the present disclosure can be modified in various ways. For example, the pharmaceutical support server 10 may be configured by a plurality of computers separated as hardware in order to improve processing capacity and reliability. For example, the functions of the receiving unit 65 and the RW control unit 66 and the functions of the feature amount derivation unit 67, the first prediction unit 68, the second prediction unit 69, and the delivery control unit 70 are distributed to two computers. In this case, the pharmaceutical support server 10 is configured by two computers.

As described above, the hardware configuration of the computer of the pharmaceutical support server 10 can be appropriately changed according to required performances, such as processing capacity, safety, and reliability. Further, not only the hardware but also an application program, such as the operation program 60, can be duplicated or can be dispersively stored in a plurality of storages in order to ensure safety and reliability.

In each of the above-described embodiments, for example, the following various processors can be used as a hardware structure of processing units performing various processes, such as the receiving unit 65, the RW control unit 66, the feature amount derivation unit 67, the first prediction unit 68, the second prediction unit 69, and the delivery control unit 70. The various processors include, for example, the CPU 52 which is a general-purpose processor executing software (operation program 60) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various processors or by a combination of two or more processors of the same type or different types (for example, a combination of a plurality of FPGAs and/or a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of these various processors.

In the technology of the present disclosure, the above-described various embodiments and/or various modification examples may be combined with each other as appropriate. In addition, the present disclosure is not limited to each of the above-described embodiments, and various configurations can be used without departing from the gist of the present disclosure. Furthermore, the technology of the present disclosure extends to a storage medium that non-temporarily stores a program, in addition to the program.

The above descriptions and illustrations are detailed descriptions of portions related to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in the above descriptions and illustrations, the description of, for example, common technical knowledge that does not need to be particularly described to enable the implementation of the technology of the present disclosure is omitted in order to avoid confusion and facilitate the understanding of portions related to the technology of the present disclosure.

In the specification, "A and/or B" is synonymous with "at least one of A or B". That is, "A and/or B" means only A, only B, or a combination of A and B. Further, in the specification, the same concept as "A and/or B" is applied to a case in which the connection of three or more matters is expressed by "and/or".

All of the publications, the patent applications, and the technical standards described in the specification are incorporated by reference herein to the same extent as each individual document, each patent application, and each technical standard are specifically and individually stated to be incorporated by reference.

## Claims

1. A pharmaceutical support device comprising:
a processor,
wherein the processor is configured to:
acquire protein information related to a protein included in a biopharmaceutical to be preserved and a plurality of prescription information items respectively related to prescriptions of a plurality of types of candidate preservation solutions which are candidates for a preservation solution for the biopharmaceutical to be preserved;
derive a plurality of feature amounts respectively related to preservation stability of the plurality of types of candidate preservation solutions on the basis of the protein information and the prescription information items;
input the feature amount to a first machine learning model such that a first score indicating the preservation stability of the candidate preservation solution is output from the first machine learning model;
acquire measurement data indicating preservation stability measured by an actual test for a selected candidate preservation solution selected from the plurality of types of candidate preservation solutions on the basis of the first score; and
input the measurement data and a feature amount of the selected candidate preservation solution among the plurality of derived feature amounts to a second machine learning model such that a second score indicating preservation stability of the selected candidate preservation solution is output from the second machine learning model.

2. The pharmaceutical support device according to claim 1,
wherein the protein information includes at least one of an amino acid sequence of the protein or a three-dimensional structure of the protein.

3. The pharmaceutical support device according to claim 1 or 2,
wherein the prescription information includes a type and concentration of each of a buffer solution, an additive, and a surfactant included in the candidate preservation solution and a hydrogen ion exponent of the candidate preservation solution.

4. The pharmaceutical support device according to any one of claims 1 to 3,
wherein the processor is configured to derive the feature amount using a molecular dynamics method.

5. The pharmaceutical support device according to claim 4,
wherein the feature amount includes at least one of a solvent accessible surface area, a spatial aggregation propensity, or a space charge map of the protein.

6. The pharmaceutical support device according to any one of claims 1 to 5,
wherein the feature amount includes an indicator showing compatibility between the protein and the additive included in the candidate preservation solution.

7. The pharmaceutical support device according to any one of claims 1 to 6,
wherein the measurement data includes at least one of aggregation analysis data of sub-visible particles of the protein in the selected candidate preservation solution, analysis data of the protein in the selected candidate preservation solution by a dynamic light scattering method, analysis data of the protein in the selected candidate preservation solution by size exclusion chromatography, or analysis data of the protein in the selected candidate preservation solution by differential scanning calorimetry.

8. The pharmaceutical support device according to any one of claims 1 to 7,
wherein the processor is configured to:
determine ranking of the candidate preservation solutions on the basis of a plurality of the first scores output for the plurality of types of candidate preservation solutions; and
present a determination result of the ranking.

9. The pharmaceutical support device according to any one of claims 1 to 8,
wherein the processor is configured to:
output a plurality of the second scores for a plurality of types of the selected candidate preservation solutions;
determine ranking of the selected candidate preservation solutions on the basis of the plurality of second scores; and
present a determination result of the ranking.

10. The pharmaceutical support device according to any one of claims 1 to 9,
wherein the processor is configured to input prescription information of the selected candidate preservation solution among the plurality of prescription information items to the second machine learning model, in addition to the measurement data and the feature amount.

11. The pharmaceutical support device according to any one of claims 1 to 10,
wherein the protein is an antibody.

12. A method for operating a pharmaceutical support device, the method comprising:
acquiring protein information related to a protein included in a biopharmaceutical to be preserved and a plurality of prescription information items respectively related to prescriptions of a plurality of types of candidate preservation solutions which are candidates for a preservation solution for the biopharmaceutical to be preserved;
deriving a plurality of feature amounts respectively related to preservation stability of the plurality of types of candidate preservation solutions on the basis of the protein information and the prescription information items;
inputting the feature amount to a first machine learning model such that a first score indicating the preservation stability of the candidate preservation solution is output from the first machine learning model;
acquiring measurement data indicating preservation stability measured by an actual test for a selected candidate preservation solution selected from the plurality of types of candidate preservation solutions on the basis of the first score; and
inputting the measurement data and a feature amount of the selected candidate preservation solution among the plurality of derived feature amounts to a second machine learning model such that a second score indicating preservation stability of the selected candidate preservation solution is output from the second machine learning model.

13. A program for operating a pharmaceutical support device, the program causing a computer to execute a process comprising:
acquiring protein information related to a protein included in a biopharmaceutical to be preserved and a plurality of prescription information items respectively related to prescriptions of a plurality of types of candidate preservation solutions which are candidates for a preservation solution for the biopharmaceutical to be preserved;
deriving a plurality of feature amounts respectively related to preservation stability of the plurality of types of candidate preservation solutions on the basis of the protein information and the prescription information items;
inputting the feature amount to a first machine learning model such that a first score indicating the preservation stability of the candidate preservation solution is output from the first machine learning model;
acquiring measurement data indicating preservation stability measured by an actual test for a selected candidate preservation solution selected from the plurality of types of candidate preservation solutions on the basis of the first score; and
inputting the measurement data and a feature amount of the selected candidate preservation solution among the plurality of derived feature amounts to a second machine learning model such that a second score indicating preservation stability of the selected candidate preservation solution is output from the second machine learning model.
